# EUROPÄISCHE PATENTSCHRIFT

(11) **EP 0 965 632 B1**
(45) Veröffentlichungstag und Bekanntmachung des Hinweises auf die Patenterteilung: **15.01.2003**
(21) Anmeldenummer: 99110605.5
(22) Anmeldetag: 02.06.1999
(51) Int. Cl.: C12M 1/02, B01F 15/02, C12M 1/24

(54) **Anordnung zur kontinuierlichen Fermentation**
Continuous fermentation system
Dispositif de fermentation en continu

(30) Priorität: 02.06.1998 DE 19824322; 25.09.1998 DE 19843857
(43) Veröffentlichungstag der Anmeldung: 22.12.1999
(73) Patentinhaber: Büchs, Jochen, Prof. Dr.-Ing., 52074 Aachen (DE)
(72) Erfinder: Büchs, Jochen, Prof.Dr.-Ing., 52074 Aachen (DE); Maier, Bernd, Dipl.-Ing., 52064 Aachen (DE); Roth, Birthe Vivian, 52064 Aachen (DE)
(74) Vertreter: Kohlmann, Kai, Dipl.-Ing.

(56) Entgegenhaltungen:
- EP-A- 0 611 821
- WO-A-93/24606
- DE-C- 822 764
- DE-C- 4 017 754
- GB-A- 1 002 987
- GB-A- 2 062 481
- US-A- 3 572 651
- US-A- 5 577 837
- US-A- 5 593 228

## Beschreibung

Die Erfindung betrifft eine Anordnung zur kontinuierlichen sterilen Fermentation von in einem Nährmedium suspendierten Mikroorganismen oder Zellen in mindestens einem Bioreaktor mit einer Versorgung für Gase und Nährmedium und einem gemeinsamen Auslass für Abgase und Kulturflüssigkeit sowie mit einer Vorrichtung zur Durchmischung der Kulturflüssigkeit, wobei die Gasversorgung als Oberflächenbegasung der Kulturflüssigkeit ausgestaltet ist.

Derartige Anordnungen werden zur Durchführung biochemischer, insbesondere aerober Reaktionen (Fermentation) im Labormaßstab, insbesondere für die Anzucht von in Nährmedium suspendierten Mikroorganismen und Zellen eingesetzt. Die suspendierten Organismen werden durch das Nährmedium mit Nährstoffen versorgt. Eine ausreichende Belüftung des Nährmediums muss bei aeroben Bioprozessen gewährleistet sein, da die Mikroorganismen und Zellen nur den in der Kulturflüssigkeit gelösten Sauerstoff verwerten können und gasförmige Stoffwechselprodukte als Abgas abgeführt werden müssen.

Aus Laborexperimenten in kontinuierlicher Kultur können wertvolle Informationen über das grundsätzliche Verhalten (z.B. Limitierungen, Hemmungen) und die Kinetik eines biologischen Systems gewonnen werden. So sind einige Parameter biologischer Modelle, wie z.B. die Monodkonstanten oder Zellerhaltungskoeffizienten, in diskontinuierlicher Kultur nur mit unzureichender Genauigkeit zu bestimmen und müssen daher in kontinuierlicher Kultur ermittelt werden.

Zur kontinuierlichen sterilen Fermentation werden in der Regel Rührfermenter als Bioreaktoren eingesetzt. Rührfermenter weisen meist geschlossene Behälter aus Edelstahl, Glas oder Kunststoff auf, die mit einer Versorgung für Gase, Nährmedium und ggf. Impflösung und einem Auslass in Form eines Abzuges für Abgase und eines weiteren Abzuges für Kulturflüssigkeit sowie mit einer Vorrichtung zur Durchmischung der Kulturflüssigkeit ausgestattet sind. Die Vorrichtung zur Durchmischung ist in der Regel ein mechanischer Rührer, der von einer mit Sterildichtungen oder einer Magnetkupplung versehenen Rührerwelle angetrieben wird. Der Sauerstoff wird zumeist submers durch ein Begasungsrohr oder einen Begasungsring in die Kulturflüssigkeit eingebracht. Als Sterilbarriere für die Gaszufuhr und die Gasabfuhr dient in der Regel ein Membranoder Tiefenfilter.

Bei kontinuierlich betriebenen Rührfermentern ist ein erheblicher apparativer und steriltechnischer Aufwand erforderlich. Nach dem Stand der Technik kann ein absolut konstantes Reaktionsvolumen bei Rührfermentern nur durch die Verwendung einer teuren Wägeeinrichtung garantiert werden. Diese funktioniert jedoch aufgrund des ungünstigen Verhältnisses der zu messenden Gewichtsänderungen in dem Bioreaktor zu der Starrheit der Zuführungsleitungen und Kabel zu dem Bioreaktor zuverlässig nur bei Bioreaktoren mit einem Reaktionsvolumen größer als etwa einem Liter. Aufgrund dieser vergleichsweise großen Reaktionsvolumina ist es notwendig, ständig große Mengen an sterilem Nährmedium bereitzustellen. Darüber hinaus ergeben sich bis zur Einstellung des Fließgleichgewichtes in den stationären Betriebspunkten (ohne zeitliche Konzentrationsänderungen) lange Fermentationszeiten. Diese können bei sukzessiver Einstellung mehrerer Fließgleichgewichte mehrere Monate betragen und machen derartige Versuche sehr kontaminationsanfällig. Bei einer Kontamination des Bioreaktors muss der Versuch abgebrochen und der gesamte vorbereitungs- und Anwachsvorgang wiederholt werden. Die obengenannten Nachteile machen die kontinuierliche Fermentationen mit den bekannten Rührfermentern sehr zeit- und arbeitsaufwendig und teuer. Aufgrund des komplizierten Aufbaus und des sehr hohen Preises von Rührfermentern ist ein paralleler Betrieb mehrerer Rührfermenter mit einem Reaktionsvolumen größer einem Liter bereits aus Kostengründen nur sehr begrenzt möglich.
Ein weiterer Nachteil von Rührfermentern ist die submerse Sauerstoffversorgung über ein Begasungsrohr oder einen Begasungsring in der Kulturflüssigkeit. Prozesse wie Blasenbildung, -rezirkulation, -koaleszenz und -abscheidung an der Flüssigkeitsoberfläche sind schwer zu beschreiben und zu kontrollieren. Unterschiedliche Zusammensetzungen des Nährmediums und dessen unweigerliche Änderungen infolge der metabolischen Aktivität der Mikroorganismen während einer Fermentation wirken sich infolgedessen zum Teil stark auf den Stofftransport aus. Somit sind Rührfermenter hinsichtlich ihrer Fläche für den Stoffaustausch zwischen Gasphase und Kulturflüssigkeit zur Absorption von Sauerstoff und Desorption von Kohlendioxid wenig definiert. Daher muss die Gelöstsauerstoffkonzentration durch eine aufwendige Messung beispielsweise durch eine pO2-Elektrode kontrolliert werden, was die Kosten weiter in die Höhe treibt. Bei den submers begasten Rührfermentern neigt die Kulturflüssigkeit auf Grund der Begasung mit Luft und der feinen Dispergierung der Gasphase zum Bilden von feinporigem, oft sehr stabilem Schaum. Die Schaumbildung ist unerwünscht, da der Schaum Kulturflüssigkeit aus dem Rührfermenter austrägt und der Abgassterilfilter dadurch verstopft. Nur durch Einsatz einer aufwendigen Schaumdetektion, beispielsweise über Leitfähigkeitsmeßsonden oder kapazitive Sonden in Verbindung mit den bekannten chemischen oder mechanischen Schaumzerstörern lässt sich die Schaumbildung beherrschen. Schließlich bilden sich bei submerser Begasung durch an der Oberfläche der Kulturflüssigkeit platzende Gasblasen Aerosole. Dies kann zu einer Kontamination der sterilen Nährmediumvorlage durch in den Aerosolen enthaltene Mikroorganismen führen, die durch die Medienzuführung in das Vorlagegefäß zurückwachsen.

Aus der GB-A-1,002,987 ist ein Bioreaktor für die kontinuierliche Kultur von lebenden Zellen bekannt, der über eine ventilgesteuerte Saugleitung mit einem Tank für die Kulturflüssigkeit verbunden ist und einen mit einem Gasdruckbehälter verbundenen Gaseinlass in dem Verschluss des Bioreaktors aufweist, über den Sauerstoff, Stickstoff und Kohlendioxid entsprechend den Bedingungen der kultivierten Zellen zugeführt wird. Eine Photosensoreinheit öffnet das den Bioreaktor mit dem Tank für die Kulturflüssigkeit verbindende Ventil bei Erreichen eines oberen Grenzwertes der Zellkonzentration im Bioreaktor und schließt das Ventil, wenn eine untere Grenze erreicht wird. Durch Öffnen des Ventils steigt der Flüssigkeitsspiegel in dem Bioreaktor bis an ein mit einem Vakuumerzeuger verbundenes, in dem Bioreaktor mündendes Saugrohr, über das die mit Zellen angereicherte Kulturflüssigkeit abgeleitet wird.

Die US-A-5,577,837 zeigt eine Rotationsschüttelmaschine für mehrere der Zellkultur dienende Bioreaktoren. Die Bioreaktoren sind nicht mit einem Auslass für Abgase und/oder Kulturflüssigkeit versehen.

Ausgehend von diesem Stand der Technik liegt der Erfindung daher die Aufgabe zu Grunde, eine Anordnung zur kontinuierlichen sterilen Fermentation der eingangs erwähnten Art zu schaffen, mit der sich Fermentationen mit konstantem Reaktionsvolumen kostengünstig parallelisieren und damit die Versuchszeiten verkürzen lassen, bei der die Sauerstoffversorgung der Mikroorganismen ohne apparativen Aufwand ausreichend definiert ist und eine Schaumbildung der Kulturflüssigkeit sowie eine Kontamination der sterilen Nährmediumvorlage vermieden wird.

Die Lösung dieser Aufgabe basiert auf dem Gedanken, den bei diskontinuierlichen Fermentationen bekannten Parallelansatz mittels geschüttelter Bioreaktoren auf die kontinuierliche Fermentation zu übertragen. Geschüttelte Bioreaktoren nach dem Stand der Technik weisen zwar das gewünschte kleine, im Bereich weniger Milliliter liegende Reaktionsvolumen auf, sind jedoch für kontinuierliche Fermentationen ungeeignet. Im einzelnen wird die Aufgabe bei einer Anordnung der eingangs erwähnten Art dadurch gelöst, dass jeder Bioreaktor auf einer Rotationsschüttelmaschine angeordnet ist, dass der Querschnitt des Bioreaktors) längs seiner Achse kreisförmig ist, die Achse des als Ablaufrohr ausgestalteten Auslasses in einem Winkel α von 90° bis 0° zu einer Tangente an die Umfangslinie des kreisförmigen Querschnitts des Bioreaktors verläuft, insbesondere in einem Winkel α von 3° bis 10° nach außen, und die Achse des Ablaufrohres in einem Winkel β gegenüber der Horizontalen nach unten geneigt verläuft, insbesondere in einem Winkel β von 2° bis 45°.

Für die Durchmischung der Kulturflüssigkeit und für einen ausreichenden Sauerstoffeintrag sowie die Entfernung des gebildeten Kohlendioxid in mehreren Bioreaktoren gleichzeitig sorgt eine handelsübliche Rotationsschüttelmaschine, die eine kreisförmige, translatorische Bewegung der auf ihrem Tablar stehenden Bioreaktoren erzeugt. Wichtig für die Erfindung ist, dass die Kulturflüssigkeit durch die kreisförmige Bewegung in Rotation gebracht wird. Der Drehsinn ist lediglich bei solchen Ausführungen der Erfindung von Bedeutung, bei denen einzelne Bestandteile in Strömungsrichtung asymmetrisch angeordnet sind. Der apparative und steriltechnische Aufwand für die Durchmischung gegenüber den bekannten Rührfermentern ist erheblich reduziert, insbesondere durch die Belegung einer Rotationsschüttelmaschine mit mehreren Bioreaktoren.

Gleichzeitig lässt sich durch diese Art der Durchmischungsvorrichtung in Verbindung mit der als Oberflächenbegasung ausgestalteten Gasversorgung das Reaktionsvolumen jedes Bioreaktors erheblich verkleinern, woraus weitere Kosten- und Handhabungsvorteile resultieren. In der Handhabung und dem Aufwand ist die Anordnung dem herkömmlichen Schüttelkolben in diskontinuierlicher Kultur vergleichbar. In Versuchen ließ sich in Folge der Parallelisierung ohne Schwierigkeiten eine Verkürzung der Versuchszeit auf weniger als ein Drittel und eine Reduzierung des Volumens des benötigten Nährmediums auf etwa 1/40 gegenüber einem herkömmlichen Rührfermenter erzielen. Insbesondere ermöglicht die Oberflächenbegasung ohne zusätzlichen apparativen Aufwand eine definierte Sauerstoffversorgung der Mikroorganismen oder der Zellen und verhindert eine Schaumbildung der Kulturflüssigkeit sowie eine Kontamination der sterilen Nährmediumvorlage. Aufgrund der definierten und berechenbaren Fläche für den Stoffaustausch zwischen Gasphase und Kulturflüssigkeit der Bioreaktoren kann durch Wahl geeigneter Betriebsparameter der Anordnung (Schüttelfrequenz, Schütteldurchmesser, Reaktionsvolumen und Reaktorgeometrie) eine bestimmte Sauerstoffversorgung der Mikroorganismen von vornherein eingestellt werden.

Der im Querschnitt kreisförmige, von unten nach oben im Durchmesser gleichbleibende oder zusammen- oder auseinanderlaufende Bioreaktor kann beispielsweise aus Metall, Kunststoff oder Glas sein und muss in einem unteren, mit Kulturflüssigkeit in Berührung kommenden Bereich aus hydrophilem Material bestehen, während der obere Bereich des Bioreaktors vorzugsweise aus hydrophobem Material besteht, damit sich kein Kondensat anlagern kann. Der untere und/oder obere Teil des Bioreaktors sollte zur optischen Kontrolle des Reaktorinnenraums aus transparentem Material bestehen. Das Reaktionsvolumen der Bioreaktoren beträgt in der Regel 2 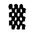 500 ml, kann aber in besonderen Anwendungsfällen bis zu 100 1 ausmachen.

Die Zufuhr für das sterile Nährmedium mündet in einer Höhe von 2 bis 20 mm über der Oberfläche der Kulturflüssigkeit bei stillstehender Rotationsschüttelmaschine. Die Mündung der Zufuhr ist zweckmäßigerweise so angeordnet, dass das Nährmedium direkt in die kreisende Kulturflüssigkeit in dem Bioreaktor tropft.

In an sich bekannter Weise ist die Gaszufuhr für jeden Bioreaktor steriltechnisch vom Innenraum des Bioreaktors getrennt. Hierzu kann beispielsweise in einen Deckel des Bioreaktors eine Sterilbarriere in Form eines Sterilfilters integriert sein.

Schließlich erlaubt die durch die kreisförmige, translatorische Bewegung des gesamten Bioreaktors erzeugte reproduzierbare Flüssigkeitsverteilung mit einer definierten Flüssigkeitsoberkante einen gemeinsamen Auslass für die Abgase und die Kulturflüssigkeit in jedem Bioreaktor vorzusehen, wobei der Abgasstrom zu einem stetigen Ablauf der Kulturflüssigkeit aus dem gemeinsamen Auslass beitragen kann. Die erfindungsgemäße Anordnung der Achse des Auslasses stellt sicher, dass die Kulturflüssigkeit einfach aus dem Reaktor austreten, jedoch nicht durch Fliehkräfte wieder in den Bioreaktor zurückgelangen kann. Unterstützen lässt sich dieser Effekt, wenn die Materialien des Ablaufrohres hydrophile und die daran angeschlossene Leitung für die Ableitung der Kulturflüssigkeit und die Abgase vorzugsweise hydrophobe Eigenschaften aufweisen. Um ein sicheres und stetiges Ablaufen der Kulturflüssigkeit aus dem Reaktorinnenraum zu gewährleisten, sollte die Mündung des Ablaufrohres im Reaktorinnenraum möglichst scharfkantig mit einem maximalen Kantenradius zwischen dem Ablaufrohr und dem Reaktorinnenraum von 0,2 mm ausgebildet sein. Das Ablaufrohr kann beispielsweise aus Metall, Glas oder Kunststoff bestehen und wird vorzugsweise in eine zuvor in den Bioreaktor eingebrachte Bohrung mittels eines sterilisierbaren Klebers eingeklebt oder im Fall einer Metallolive mittels O-Ringen abgedichtet. Wird der Bioreaktor und das Ablaufrohr aus Quarzglas gefertigt, kann statt einer Verklebung das Ablaufrohr an der Reaktoraußenseite mit dem Bioreaktor verschweißt werden.

Mit Hilfe der erfindungsgemäßen Anordnung ist es erstmals gelungen, mit vertretbarem Aufwand in großem Umfang kontinuierliche Fermentationen parallel zu betreiben. So können mit unterschiedlichen Bioreaktoren gleichzeitig verschiedene Betriebspunkte eingestellt werden, so dass z.B. schon nach einmaligem Einstellen des Fließgleichgewichtes bereits die Daten für ein vollständiges X-D-Diagramm vorliegen. Dabei ist mit Betriebspunkt jeweils ein Fließgleichgewicht bei einer bestimmten Durchflussrate gemeint. Die Dauer zur Einstellung eines Fließgleichgewichtes ist etwa 5-20 mal so lang wie die mittlere Verweilzeit der Kulturflüssigkeit in dem Bioreaktor.

Durch die Parallelisierung lässt sich im Vergleich zu Anordnungen mit einem Rührfermenter der experimentelle Zeitaufwand erheblich verkürzen. Gibt es in einem der erfindungsgemäßen Bioreaktoren Störungen, so fehlen lediglich die Daten für einen Betriebspunkt. Bei der Verwendung von nur einem Rührfermenter muss dagegen der gesamte Vorbereitungs- und Anwachsvorgang wiederholt werden.

Aus dem Stand der Technik ist es bekannt, bei biologischen Prozessen, die beispielsweise eine Produktinhibierung aufweisen, kontinuierliche Bioreaktoren, z.B. mehrere Rührfermenter, in einer Kaskade hintereinanderzuschalten. Außerdem ist es bekannt, bei Prozessen, die eine sogenannte Katabolitrepression aufweisen, zusätzlich mittels einer Zwischeneinspeisung auch den weiter hinten befindlichen Bioreaktoren der Kaskade Nährmedium zuzuführen.

Gemäß der Erfindung ist es möglich, eine vollständige Kaskade mit mehreren Bioreaktoren durch einen entsprechend den Merkmalen des Anspruchs 3 ausgestalteten kaskadierten Bioreaktor zu ersetzen, von denen sich mehrere mit Hilfe der erfindungsgemäßen Anordnung ohne großen Aufwand parallel betreiben lassen. Das eröffnet ganz neue Möglichkeiten bei der reaktionstechnischen Erfassung des Verhaltens von mikrobiellen Kulturen und bei der Entwicklung von Bioprozessen. Die zur Unterteilung erforderlichen, im Querschnitt kreisförmigen Einbauten, können von unten nach oben im Durchmesser gleichbleiben oder zusammen- oder auseinanderlaufen.

Eine Kurzschlussströmung zwischen den Reaktorstufen eines kaskadierten Bioreaktors kann nach den Merkmalen des Anspruchs 6 dadurch wirksam verhindert werden, dass die Einschnitte oder die Bohrungen benachbarter Einbauten sowie die zum Ablaufrohr benachbarten Einschnitte oder Bohrungen in Drehrichtung der Kulturflüssigkeit möglichst weit versetzt zueinander angeordnet sind, ohne sich jedoch zu überlappen. Hierzu sind die Einschnitte oder die Bohrungen und das Ablaufrohr auf entgegengesetzten Seiten, bevorzugt jedoch etwas entgegen der Drehrichtung der Kulturflüssigkeit versetzt zueinander angeordnet. Zusätzlich sollten die Überlaufhöhen zwischen den Reaktorstufen von innen nach außen leicht ansteigen, um Kurzschlussströmungen zu verhindern.

Wenn die konzentrischen Einbauten und/oder das Ablaufrohr des kaskadierten Bioreaktors austauschbar sind, lassen sich abhängig von der gewünschten Reaktionsführung unterschiedliche Überlaufhöhen und -querschnitte einstellen. Durch diese Maßnahme lassen sich unterschiedliche Volumenverhältnisse in den Reaktorstufen realisieren.

Nachfolgend wird die Erfindung anhand der Figuren 1 bis 7 näher erläutert. Es zeigen;
- Figur 1: eine schematische Darstellung einer erfindungsgemäßen Anordnung zur kontinuierlichen Fermentation;
- Figur 2a: ein erstes Ausführungsbeispiel eines Bioreaktors zum Einsatz in einer Anordnung nach Figur 1;
- Figur 2b: eine geschnittene Darstellung eines Details des Bioreaktors nach Figur 2a;
- Figur 2c: eine geschnittene Darstellung einer alternativen Ausführung des Details nach Figur 2b;
- Figur 3: ein zweites Ausführungsbeispiel eines Bioreaktors zum Einsatz in einer Anordnung nach Figur 1;
- Figur 4: ein drittes Ausführungsbeispiel eines Bioreaktors zum Einsatz in einer Anordnung nach Figur 1;
- Figur 5a: zwei Schnittdarstellungen eines ersten Ausführungsbeispiels eines kaskadierten Bioreaktors zum Einsatz in einer Anordnung nach Figur 1;
- Figur 5b: eine abgewickelte Darstellung eines Details des kaskadierten Bioreaktors nach Figur 5a in Außenansicht;
- Figur 6: zwei Schnittdarstellungen eines zweiten Ausführungsbeispiels eines kaskadierten Bioreaktors zum Einsatz in einer Anordnung nach Figur 1;
- Figur 7a: zwei Schnittdarstellungen eines dritten Ausführungsbeispiels eines kaskadierten Bioreaktors zum Einsatz in einer Anordnung nach Figur 1 sowie
- Figur 7b: eine geschnittene Darstellung eines Details des Bioreaktors nach Figur 7a.

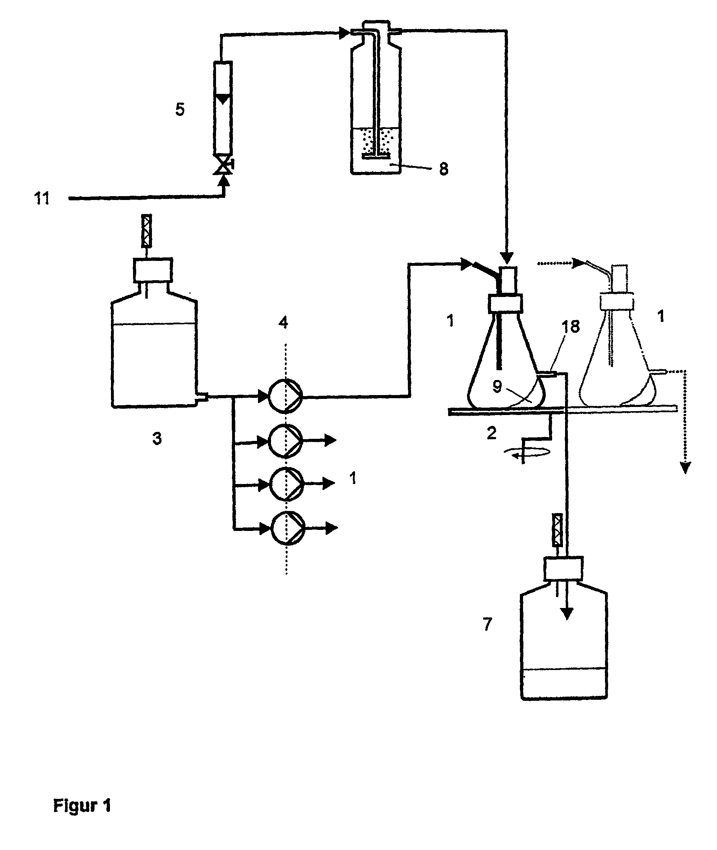

Die erfindungsgemäße Anordnung zur kontinuierlichen Fermentation nach **Figur 1** besteht im wesentlichen aus mehreren Bioreaktoren (1), die auf einer Rotationsschüttelmaschine (2) angeordnet sind. Sämtliche auf einer Rotationsschüttelmaschine (2) angeordnete Bioreaktoren (1) werden im Ausführungsbeispiel aus einem gemeinsamen Vorlagegefäß (3) über eine Mehrkanalschlauchpumpe (4) kontinuierlich mit sterilem Nährmedium versorgt. Die Versorgung aus einen gemeinsamen Vorlagegefäß (3) ist allerdings nicht zwingend. Durch Verändern des Durchmessers der Pumpenschläuche und/oder der Pumpendrehzahl lässt sich der Volumenstrom des Nährmediums für jeden Bioreaktor (1) individuell anpassen.

Zur Begasung erforderliches Gas wird für jeden Bioreaktor (1) von einem Druckgasanschluss (11) über einen Volumenstromregler (5) und eine zur Befeuchtung des Gases dienende Gaswaschflasche (6) geleitet. Es ist jedoch auch möglich, eine zentrale Gaswaschflasche für mehrere Bioreaktoren zu verwenden, während die Volumenstromregler (5) in jedem Fall für jeden Bioreaktor separat vorgesehen sein sollten. Zur Einstellung der gleichen Wasseraktivität wie in der Kulturflüssigkeit (9) sind die Gaswaschflaschen (6) mit einer entsprechenden Salzlösung (8) gefüllt. Das Abgas und die Kulturflüssigkeit (9) werden durch einen gemeinsamen als Ablaufrohr (18) ausgestalteten Auslas im Bioreaktor (1) in ein Erntegefäß (7) abgeleitet, wobei der Abgasstrom zu einem stetigen Ablauf der Kulturflüssigkeit (9) aus dem Ablaufrohr (18) beiträgt.

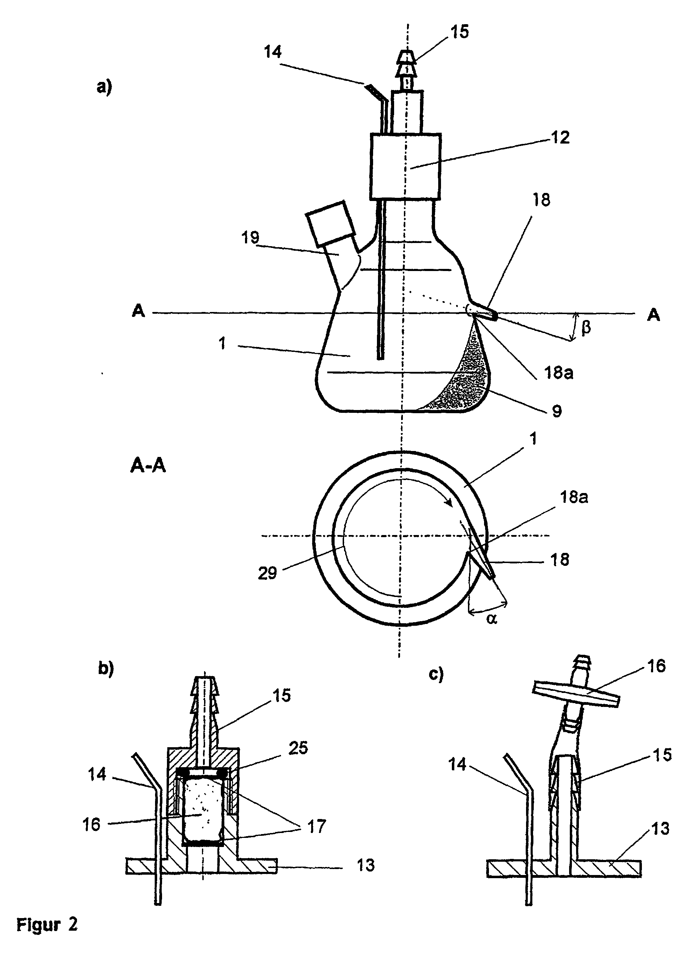

Der in **Figur 2** dargestellte, im Querschnitt kreisförmige Bioreaktor (1) kann aus Kunststoff, Glas oder Metall gefertigt sein; allerdings sind transparente Materialien zu bevorzugen. Die für die erfindungsgemäße Anordnung konzipierten Bioreaktoren (1) werden durch einen an der oberen Stirnseite des Bioreaktors (1 angeordneten Deckel (12) mit Nährmedium und Gas versorgt. In dem Deckel (12) befindet sich hierfür ein Deckeleinsatz (Figur 2b) mit einer Grundplatte (13) durch die ein Rohr (14) für die Zufuhr des Nährmediums geführt ist. Das Rohr (14) endet in einer definierten Höhe über dem Flüssigkeitsspiegel der ruhenden Kulturflüssigkeit (9), so dass das zugeführte Nährmedium bei den durch die Schüttelfrequenz und den Schütteldurchmesser gegebenen Fliehkraftverhältnissen direkt in die im Bioreaktor (1) kreisende Kulturflüssigkeit (9) tropft. in Versuchen hat sich herausgestellt, dass das Rohr (14) vorteilhafter Weise in einer Höhe von 2 bis 20 mm über der ruhenden Kulturflüssigkeit (9) endet. Der Innendurchmesser des Rohres (14) soll so bemessen sein, dass die darin enthaltene Flüssigkeit nicht selbständig ausläuft. In Versuchen haben sich für den Innendurchmesser des Rohres (14) Werte im Bereich von 1/20 bis 1/200, vorzugsweise jedoch im Bereich von 1/60 bis 1/100 des maximalen Innendurchmessers des Bioreaktors (1) als praktikabel herausgestellt.

Der Gasstrom wird dem Bioreaktor (1) über einen Schlauchanschluss (15) und einen Sterilfilter (16), der durch eine Lochplatte (17) vom Reaktorinnenraum getrennt ist, zugeführt. Der Schlauchanschluss (15) ist lösbar, jedoch über einen O-Ring (25) dichtend, auf einen Stutzen der Grundplatte (13) des Deckels (12) aufgeschraubt. An seiner Innenmantelfläche besitzt der Stutzen eine umlaufende, ringförmige Schulter, auf der der Rand der Lochplatte (17) aufliegt. Oberhalb der Lochplatte befindet sich der Sterilfilter (16), der durch Abschrauben des Schlauchanschlusses (15) zugänglich ist und aus dem Stutzen der Grundplatte (13) entfernt und ausgetauscht werden kann. In einer besonders einfachen Ausführung nach Figur 2c ist der Schlauchanschluss (15) unlösbar mit der Grundplatte (13) verbunden und es kann ein kommerziell erhältlicher Sterilfilter (16) als Sterilbarriere eingesetzt werden, der in die Schlauchleitung eingesetzt wird.

Durch Anordnung des Ablaufrohres (18) in unterschiedlichen Höhen können in bei sonst gleichen Betriebsparametern betriebenen Bioreaktoren (1) unterschiedliche Füllmengen der Kulturflüssigkeit (9) und damit unterschiedliche Durchflussraten eingestellt werden.

Das Ablaufrohr (18) ist so gestaltet, dass die Kulturflüssigkeit (9) problemlos aus dem Bioreaktor (1) austreten, jedoch nicht durch Fliehkräfte wieder in den Bioreaktor (1) zurückgelangen kann. Das wird durch eine zum kreisförmigen Bioreaktor (1) vorzugsweise annähernd tangentiale (vgl. den Winkel α in Figur 2a) und gegenüber der Horizontalen um den Winkel β nach unten gerichtete Orientierung der Achse des Ablaufrohres (18a) erreicht. Um ein sicheres und stetiges Ablaufen der Kulturflüssigkeit (9) aus dem Reaktorinnenraum zu gewährleisten, sollte die Mündung (18a) des Ablaufrohres (18) im Reaktorinnenraum möglichst scharfkantig, mit einem maximalen Kantenradius zwischen dem Ablaufrohr (18) und dem Reaktorinnenraum von 0,2 mm, ausgebildet sein. Ein störungsfreier Austritt der Kulturflüssigkeit (9) aus dem im Querschnitt kreisförmigen Bioreaktor (1) ist gewährleistet, wenn nachfolgende Bedingungen für die Orientierung des Ablaufrohrs (18) eingehalten werden:
- die Achse des Ablaufrohres (18) verläuft in einem Winkel α von 90° bis 0° zu einer Tangente an die Umfangslinie des kreisförmigen Querschnitts, vorzugsweise beträgt der Winkel α jedoch ca. 3° bis 10° und
- die Achse des Ablaufrohres (18) verläuft in einem Winkel β gegenüber der Horizontalen nach unten geneigt, insbesondere in einem Winkel β von 2° bis 45°.

Der Innendurchmesser des Ablaufrohres (18) beträgt vorzugsweise zwischen 1/25 bis 1/100 des maximalen Innendurchmessers des Bioreaktors (1).

Zum Animpfen der Kultur und zur Probenahme sind die Bioreaktoren (1) mit einem verschließbaren Stutzen (19) ausgestattet.

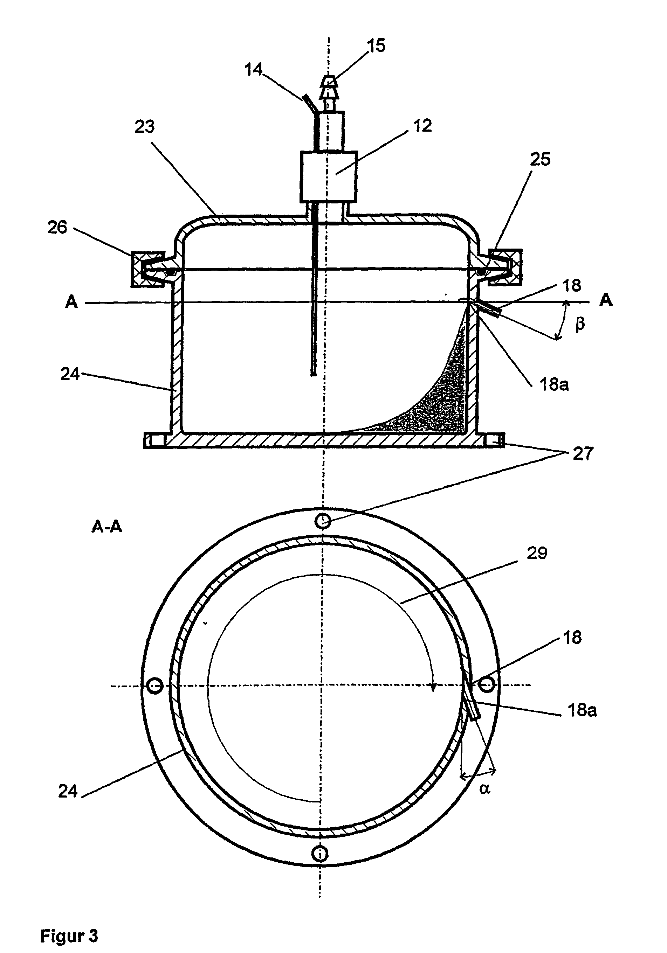

In **Figur 3** ist eine zweiteilige Ausführung eines kontinuierlichen Bioreaktors (1) gezeigt. Der Bioreaktor (1) ist in Längsrichtung in ein Oberteil (23) und ein Unterteil (24) aufgeteilt. Zwischen den mittels einer Schelle (26) verbundenen Stoßstellen von Ober- und Unterteil (23,24) befindet sich ein O-Ring (25), um den Bioreaktor (1) steril abzudichten. Der Bioreaktor (1) ist mittels eines Bohrungen (27) aufweisenden Bodenflansches leicht auf der Rotationsschüttelmaschine (2) zu befestigen.

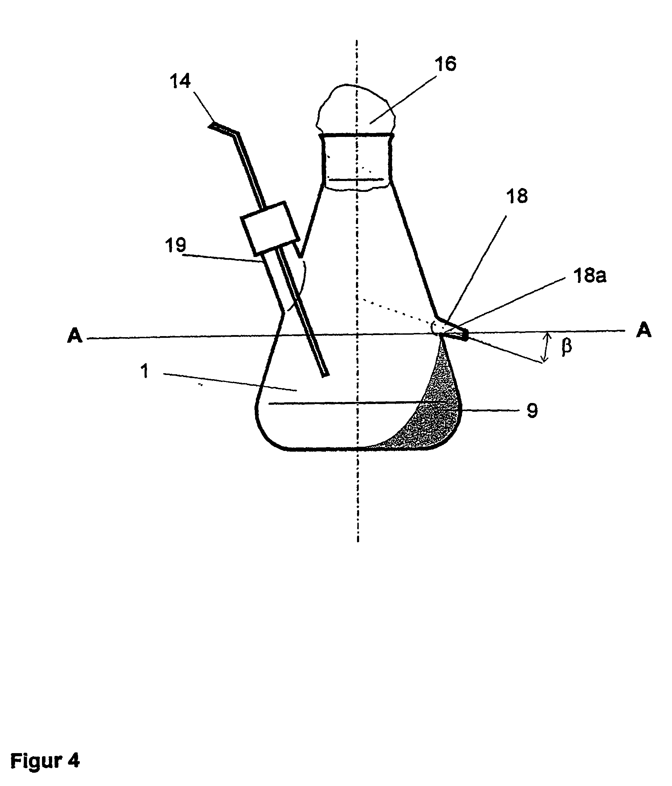

In **Figur 4** ist eine besonders einfache und daher preiswerte Variante des kontinuierlichen Bioreaktors (1) gezeigt. Hier wurde die in Figur 1 dargestellte Begasungsstrecke umfassend den Volumenstromregler (5) und die Gaswaschflasche (6), wie bei konventionellen, diskontinuierlich betriebenen Schüttelreaktoren üblich, durch einen Wattestopfen als Sterilfilter (16) ersetzt. Der Wattestopfen lässt eine ausreichende Versorgung der mikrobiellen Kultur mit Luft zu. Die Zufuhr des Nährmediums erfolgt bei dieser einfachen Variante ebenfalls über ein Rohr (14), das allerdings abweichend von den in Figuren 1,2 und 3 gezeigten Ausführungsbeispielen durch den seitlichen Stutzen (19) geführt ist.

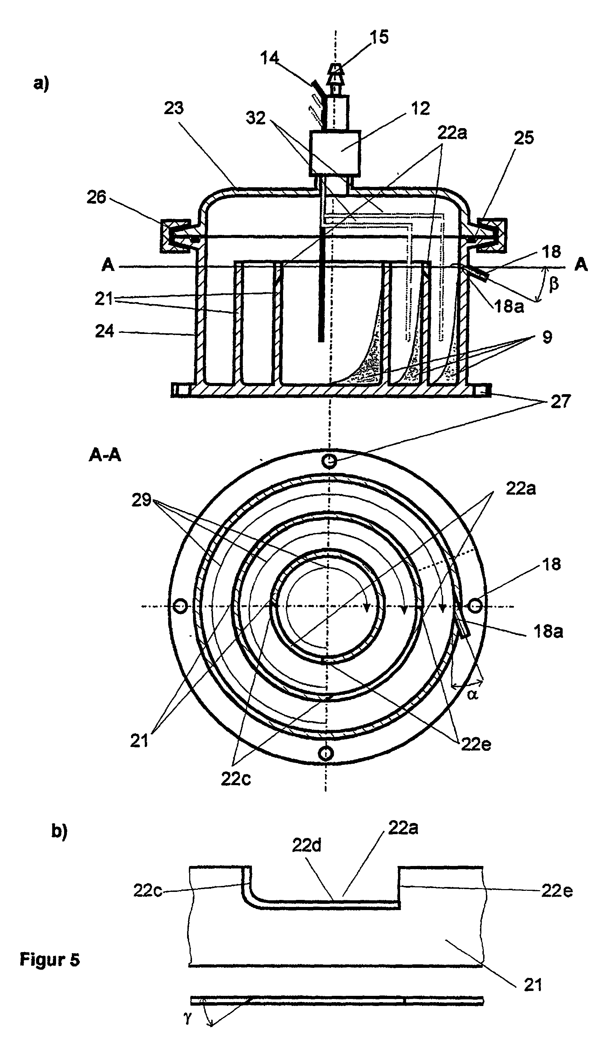

In **Figur 5** ist eine kaskadierte Ausführung des kontinuierlichen Bioreaktors (1) dargestellt. Konzentrisch zur Längsachse angeordnete, im Querschnitt kreisförmige Einbauten (21) unterteilen den Bioreaktor (1) in mehrere Reaktorstufen. In dem Ausführungsbeispiel ist der Bioreaktor (1) in Längsrichtung in ein Oberteil (23) und ein Unterteil (24) aufgeteilt. Zwischen den mittels einer Schelle (26) verbundenen Stoßstellen von Ober- und Unterteil (23,24) befindet sich ein O-Ring (25), um den Bioreaktor steril abzudichten. Der Bioreaktor (1) ist mittels eines Bohrungen (27) aufweisenden Bodenflansches leicht auf der Rotationsschüttelmaschine (2) zu befestigen.

In den als Trennwände ausgebildeten Einbauten (21) zwischen den unterschiedlichen Reaktorstufen des Bioreaktors (1) sind an deren oberen Rand relativ breite Einschnitte (22a) über einen Umfangswinkel von etwa 90° als Überlauf für die Kulturflüssigkeit (9) angebracht. Die Einschnitte (22a) sind an ihren in Strömungsrichtung (29) der Kulturflüssigkeit (9) liegenden Stirnseiten (22c) und an ihren Unterseiten (22d) scharfkantig mit einem Schneidenwinkel γ kleiner 40° ausgeführt (Figur 5b). Die stirnseitige Kante (22e) entgegen der Strömungsrichtung (29) kann dagegen stumpf ausgeführt sein.

Eine Kurzschlussströmung zwischen benachbarten Reaktorstufen des kaskadierten Bioreaktors (1) kann dadurch wirksam verhindert werden, dass die Tiefe der Einschnitte (22a) von der Mitte des Bioreaktors (1) nach außen hin abnimmt, wodurch die Überlaufhöhen der Reaktorstufen von innen nach außen leicht ansteigen, und die Einschnitte (22a) in Strömungsrichtung (29) der Kulturflüssigkeit (9) möglichst weit versetzt zueinander angeordnet sind, ohne sich jedoch zu überlappen. Die in Strömungsrichtung hintere Kante (22e) des Einschnittes (22a) liegt dann der vorderen (scharfkantigen) Kante (22c) des weiter außen gelegenen, benachbarten Einschnitts (22a) bzw. des Ablaufrohres (18) gegenüber.

In gestrichelter Form sind außerdem in Figur 5 a) noch Zwischeneinspeisungen (32) für Nährmedium für die beiden äußeren Reaktorstufen dargestellt. Somit können sogar katabolitreprimierte Prozesse mit dem kontinuierlichen Bioreaktor (1) bearbeitet werden.

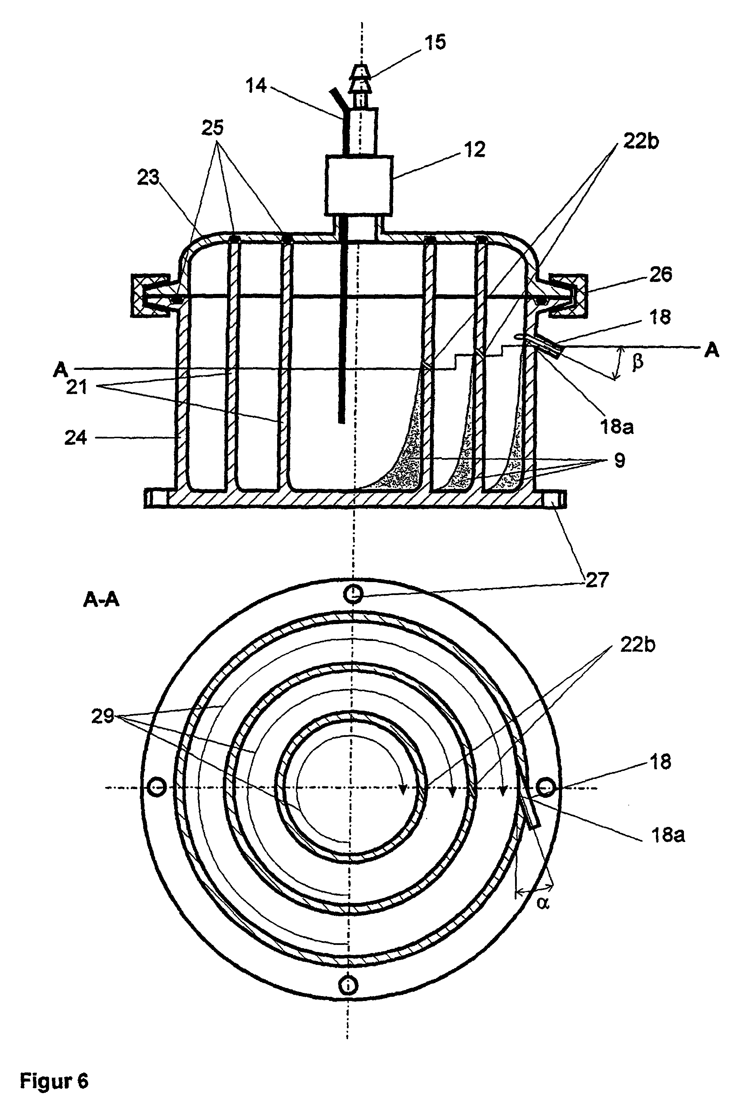

In **Figur 6** ist eine Variante des kaskadierten Bioreaktors (1) dargestellt, bei der sich die konzentrischen Einbauten (21) allerdings über die volle Höhe des Bioreaktors (1) erstrecken und bis an das Oberteil (23) heranreichen. O-Ringe (25) dichten die Einbauten (21) gegen das Oberteil (23) ab. In den als Trennwänden ausgebildeten Einbauten (21) sind zu deren kreisförmigen Querschnitten annähernd tangentiale und gegenüber der Horizontalen (1) nach unten geneigte Bohrungen (22b) angebracht. Diese Anordnung hat den Vorteil, dass der Gasstrom den Flüssigkeitstransport zwischen den Reaktorstufen innerhalb des Bioreaktors (1) unterstützt.

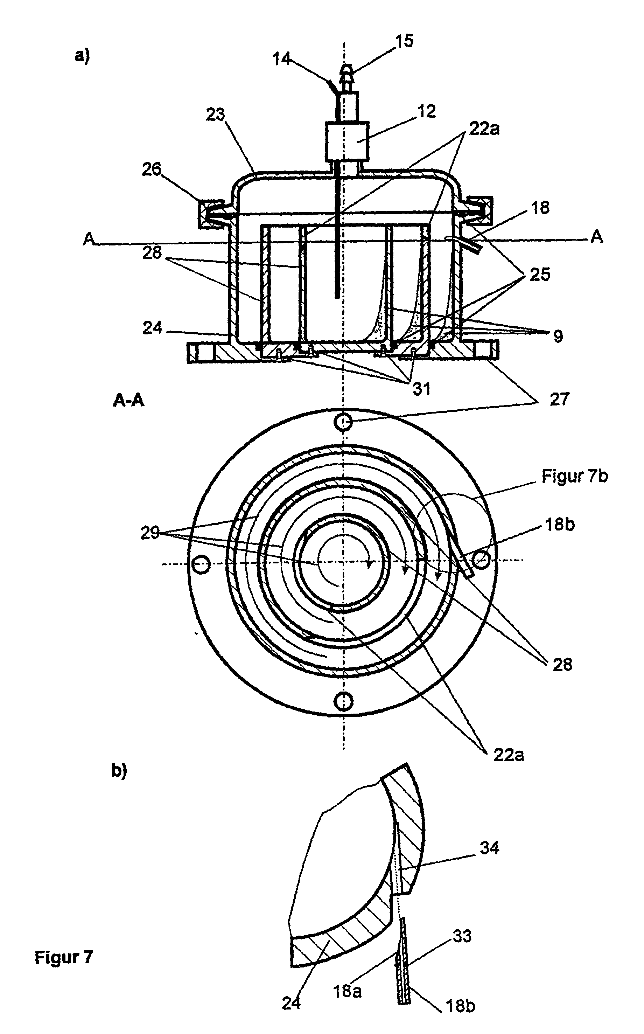

Der modulare Aufbau des in **Figur 7** dargestellten Bioreaktors (1) erlaubt den Austausch der als Gefäße (28) ausgebildeten konzentrischen Einbauten sowie des Ablaufrohres (18b). Hierdurch lassen sich abhängig von der gewünschten Reaktionsführung unterschiedliche Überlaufhöhen und/oder -querschnitte einstellen. Das austauschbare Ablaufrohr (18b) besteht aus einem auf der Innenseite entsprechend der Innenkontur des Bioreaktors (1) angeschliffenen Rohr und wird mittels eines O-Ringes (33) gegen die annähernd tangentiale und gegenüber der Horizontalen nach unten geneigte Bohrung (34) abgedichtet. Durch das Anbringen von mehreren Bohrungen (34) in unterschiedlichen Höhen kann das Reaktionsvolumen der äußeren Reaktorstufe durch Einstecken des Ablaufrohres (18b) in der gewünschten Höhe verändert werden. Nicht benötigte Bohrungen (34) werden durch einen wiederum auf der Innenseite entsprechend der Innenkontur des Bioreaktors (1) angeschliffenem Stift verschlossen. Selbstverständlich lässt sich auch ein einstufiger Bioreaktor, wie er in den Figuren 1 bis 4 dargestellt ist, mit austauschbaren Ablaufrohren ausstatten, um unterschiedliche Reaktionsvolumen einstellen zu können.

Das Unterteil (24) des modular aufgebauten Bioreaktors (1) nimmt mehrere der austausch- und ineinandersteckbaren topfartigen Gefäße (28) auf, die mittels O-Ringen (25) bodenseitig gegeneinander abgedichtet und durch Schrauben (31) oder mittels einer Presspassung miteinander verbunden sein können. Um die Schraubverbindung zwischen den Gefäßen von der Unterseite des Bioreaktors zu ermöglichen, weist zumindest das Unterteil 24 (bei lediglich 2 Gefäßen ausreichend) eine zur Achse des Bioreaktors (1) vorzugsweise konzentrische, kreisförmige Aussparung auf, die die Schraubverbindung zwischen den beiden Gefäßen von der Unterseite zugänglich macht. Durch Austausch der Gefäße (28) gegen eine das Unterteil (24) verschließende Grundplatte ist es möglich, den Bioreaktor nicht kaskadiert zu betreiben.

### Bezugszeichenliste:

| **Nr.** | **Bezeichnung** |
|---|---|
| 1 | Bioreaktor |
| 2 | Rotationsschüttelmaschine |
| 3 | Vorlagegefäß |
| 4 | Mehrkanalschlauchpumpe |
| 5 | volumenstromregler |
| 6 | Gaswaschflasche |
| 7 | Erntegefäß |
| 8 | Salzlösung |
| 9 | Kulturflüssigkeit |
| 10 | ------------------- |
| 11 | Druckgasanschluss |
| 12 | Deckel |
| 13 | Grundplatte |
| 14 | Rohr |
| 15 | Schlauchanschluss |
| 16 | Sterilfilter |
| 17 | Lochplatte |
| 18 | Ablaufrohr |
| 18a | Scharfkantige Mündung |
| 18b | Austauschbares Ablaufrohr |
| 19 | Stutzen |
| 20 | ------------------- |
| 21 | konzentrische Einbauten |
| 22a | Einschnitt |
| 22b | Bohrung |
| 22c | Scharfe Vorderkante |
| 22d | Scharfe Unterkante |
| 22e | Stumpfe Hinterkante |
| 23 | Oberteil des Bioreaktors |
| 24 | Unterteil des Bioreaktors |
| 25 | O-Ring |
| 26 | Schelle |
| 27 | Bohrung |
| 28 | Gefäß |
| 29 | Strömungsrichtung |
| 30 | ------------------- |
| 31 | Schrauben |
| 32 | Zwischeneinspeisung |
| 33 | O-Ring Ablaufrohr 18 b |
| 34 | Bohrung für Ablaufrohr 18 b |

## Patentansprüche

1. Anordnung zur kontinuierlichen sterilen Fermentation von in einem Nährmedium suspendierten Mikroorganismen oder Zellen in mindestens einem Bioreaktor mit einer Versorgung für Gase und Nährmedium und einem gemeinsamen Auslass für Abgase und Kulturflüssigkeit sowie mit einer Vorrichtung zur Durchmischung der Kulturflüssigkeit, wobei die Gasversorgung als Oberflächenbegasung der Kulturflüssigkeit ausgestaltet ist, **dadurch gekennzeichnet,**
- **dass** jeder Bioreaktor (1) auf einer Rotationsschüttelmaschine (2) angeordnet ist,
- **dass** der Querschnitt des Bioreaktors (1) längs seiner Achse kreisförmig ist,
- die Achse des als Ablaufrohr (18) ausgestalteten Auslasses in einem Winkel α von 90° bis 0° zu einer Tangente an die Umfangslinie des kreisförmigen Querschnitts des Bioreaktors (1) verläuft, insbesondere in einem Winkel α von 3° bis 10° nach außen, und
- die Achse des Ablaufrohres (18) in einem Winkel β gegenüber der Horizontalen nach unten geneigt verläuft, insbesondere in einem Winkel β von 2° bis 45°.

2. Anordnung nach Anspruch 1, **dadurch gekennzeichnet, dass** die Mündung des Ablaufrohres in den Innenraum des Bioreaktors (1) einen maximalen Kantenradius zwischen dem Ablaufrohr und dem Innenraum von 0,2 mm aufweist.

3. Anordnung nach Anspruch 2, **dadurch gekennzeichnet, dass** konzentrisch zur Achse des Bioreaktors (1) angeordnete, im Querschnitt kreisförmige Einbauten (21) den Bioreaktor (1) in mehrere Reaktorstufen unterteilen.

4. Anordnung nach Anspruch 3, **dadurch gekennzeichnet, dass** die als Trennwände ausgebildeten Einbauten (21) zwischen den unterschiedlichen Reaktorstufen des Bioreaktors (1) an deren oberen Rand Einschnitte (22a) über einen Umfangswinkel zwischen 5° - 180 °, vorzugsweise jedoch 30° - 60°, als Überlauf für die Kulturflüssigkeit (9) aufweisen.

5. Anordnung nach Anspruch 3, **dadurch gekennzeichnet, dass** die konzentrischen Einbauten (21) sich in Achsrichtung über die volle Höhe des Bioreaktors (1) erstrecken und die Reaktorstufen ausschließlich über Bohrungen (22b) miteinander verbunden sind, deren Achsen senkrecht bis tangential zu einer Tangente an die Umfangslinie des kreisförmigen Querschnitts des Bioreaktors und gegenüber der Horizontalen nach unten geneigt verlaufen.

6. Anordnung nach Anspruch 4 oder 5, **dadurch gekennzeichnet, dass** die Einschnitte (22a) oder die Bohrungen (22b) benachbarter Einbauten (21) sowie die zum Ablaufrohr benachbarten Einschnitte (22a) oder Bohrungen (22b) in Drehrichtung (29) der Kulturflüssigkeit (9) möglichst weit versetzt zueinander angeordnet sind, ohne sich jedoch zu überlappen.

7. Anordnung nach einem der Ansprüche 3 bis 6, **dadurch gekennzeichnet, dass** das Ablaufrohr (18b) des kaskadierten Bioreaktors (1) austauschbar ist und/oder der kaskadierte Bioreaktor (1) in Achsrichtung in ein Oberteil (23) und ein dichtend damit verbundenes Unterteil (24) aufgeteilt ist, das mehrere lösbar ineinandergesetzte, zumindest bodenseitig gegeneinander abgedichtete Gefäße (28) aufnimmt.

8. Anordnung nach einem der Ansprüche 1 bis 7, **dadurch gekennzeichnet, dass** mehrere auf einer Rotationsschüttelmaschine (2) angeordnete Bioreaktoren (1) von einer gemeinsamen Pumpe, vorzugsweise einer Mehrkanalschlauch pumpe(4), aus einem gemeinsamen Vorlagegefäß (3) mit Nährmedium versorgt werden.

9. Bioreaktor für eine Anordnung nach einem der Ansprüche 1 bis 8 mit einer Versorgung für Gase, Nährmedium und einem gemeinsamen Auslass für Abgase und Kulturflüssigkeit, wobei die Versorgung für Gase als eine Oberflächenbegasung der Kulturflüssigkeit ausgestaltet ist, **dadurch gekennzeichnet, dass**
- der Querschnitt des Bioreaktors (1) längs seiner Achse kreisförmig ist,
- die Achse des als Ablaufrohr (18) ausgestalteten Auslassest in einem Winkel α von 90° bis 0° zu einer Tangente an die Umfangslinie des kreisförmigen Querschnitts des Bioreaktors (1) verläuft, insbesondere in einem Winkel α von 3° bis 10° nach außen, und
- die Achse des Ablaufrohres (18) in einem Winkel β gegenüber der Horizontalen nach unten geneigt verläuft, insbesondere in einem Winkel β von 2° bis 45°.

## Claims

1. An arrangement for continuous sterile fermentation of microorganisms or cells suspended in a nutrient medium in at least one bioreactor with an inlet for gases and a nutrient medium and a joint outlet for waste gases and culture fluid as well as a device for mixing the culture fluid, wherein gas is supplied onto the surface of the culture fluid, **characterized in that**
- each bioreactor (1) is arranged on a rotary shaker machine (2),
- the cross-section of the bioreactor (1) is circular along its axis,
- the axis of the outlet provided in the form of an outlet pipe (18) runs at an angle α between 90° and 0° with respect to a tangent with the circumferential line of the circular cross-section of the bioreactor (1), in particular at an angle α between 3° and 10° towards the outside, and
- the axis of the outlet pipe (18) runs downwards at an angle β with respect to the horizontal, in particular at an angle β between 2° and 45°.

2. The arrangement in accordance with Claim 1, **characterized in that** the. opening of the outlet pipe into the interior of the bioreactor (1) has a maximum edge radius of 0.2 mm between the outlet pipe and the interior.

3. The arrangement in accordance with Claim 2, **characterized in that** structures (21) with a circular cross-section which are disposed concentrically to the axis of the bioreactor (1) divide the bioreactor (1) into a plurality of reactor stages.

4. The arrangement in accordance with Claim 3, **characterized in that** the structures (21) in the form of dividing walls between the different reactor stages of the bioreactor (1) are provided, on their upper edge, with cutouts (22a) over a circumferential angle between 5° and 180°, although preferably between 30° and 60°, which allow the culture fluid (9) to overflow.

5. The arrangement in accordance with Claim 3, **characterized in that** the concentric structures (21) extend, in the axial direction, over the full height of the bioreactor (1) and wherein the reactor stages are exclusively connected with each other by means of bores (22b) whose axes run at angle between perpendicular to and tangentially with a tangent of the circumferential line of the circular cross-section of the bioreactor as well as running downwards at an angle opposite from the horizontal.

6. The arrangement in accordance with Claims 4 or 5, **characterized in that** the cutouts (22a) or bores (22b) of adjacent structures (21) as well as the cutouts (22a) or bores (22b) adjacent to the outlet pipe are arranged, in the direction of rotation (29) of the culture fluid (9), in such a manner that they are spaced as far apart from each other as possible, although without overlapping.

7. The arrangement in accordance with one of Claims 3 through 6, **characterized in that** the outlet pipe (18b) of the cascade bioreactor (1) is exchangeable and/or the cascade bioreactor (1) is divided, in the axial direction, into an upper part (23) and a lower part (24) connected therewith in a sealing manner which receives a plurality of vessels (28) inserted into each other in a removable manner and which seal each other off at least on the bottom side.

8. The arrangement in accordance with one of Claims 1 through 7, **characterized in that** a plurality of bioreactors (1) disposed on a rotary shaker machine (2) are supplied with a nutrient medium by means of a joint pump, preferably a multichannel hose pump (4), from a joint supply vessel (3).

9. A bioreactor for an arrangement in accordance with one of Claims 1 through 8, comprising an inlet for gases and a nutrient medium and a joint outlet for waste gases and culture fluid, wherein gas is supplied onto the surface of the culture fluid, **characterized in that**
- the cross-section of the bioreactor (1) is circular along its axis,
- the axis of the outlet provided in the form of an outlet pipe (18) runs at an angle α between 90° and 0° with respect to a tangent with the circumferential line of the circular cross-section of the bioreactor (1), in particular at an angle α between 3° and 10° towards the outside, and
- the axis of the outlet pipe (18) runs downwards at an angle β with respect to the horizontal, in particular at an angle β between 2° and 45°.

## Revendications

1. Dispbsitif pour la fermentation stérile continue de microorganismes ou de cellules en suspension dans un milieu nutritif dans au moins un bioréacteur avec une alimentation pour des gaz et un milieu nutritif et une sortie commune pour les gaz brûlés et le liquide de culture et avec un dispositif pour le mélange du liquide de culture, l'alimentation en gaz étant conçue comme un gazage de surface du liquide de culture, **caractérisé,**
- **en ce que** chaque bioréacteur (1) est disposé sur une secoueuse à rotation (2),
- **en ce que** la section du bioréacteur (1) est circulaire le long de son axe,
- l'axe de la sortie conçue comme un tuyau d'écoulement (18) forme un angle α de 90° à 0° par rapport à une tangente à la ligne périphérique de la section circulaire du bioréacteur (1), en particulier un angle α de 3° à 10° vers l'extérieur et
- l'axe du tuyau d'écoulement (18) est agencé de façon inclinée vers le bas en formant un angle α par rapport à l'horizontale, en particulier en formant un angle β de 2° à 45°.

2. Dispositif selon la revendication 1, **caractérisé en ce que** le débouché du tuyau d'écoulement dans le compartiment intérieur du bioréacteur (1) présente un rayon d'arête maximum entre le tuyau d'écoulement et le compartiment intérieur de 0,2 mm.

3. Dispositif selon la revendication 2, **caractérisé en ce que** les insertions (21) de section circulaire et disposées de façon concentrique par rapport à l'axe du bioréacteur (1) subdivisent le bioréacteur (1) en plusieurs étages de réacteur.

4. Dispositif selon la revendication 3, **caractérisé en ce que** les insertions (21) conçues comme des parois de séparation présentent entre les différents étages du réacteur du bioréacteur (1) sur leur bord supérieur des entailles (22a) en formant un angle périphérique compris entre 5° et 180°, mais de préférence 30° à 60°, comme trop-plein pour le liquide de culture (9).

5. Dispositif selon la revendication 3, **caractérisé en ce que** les insertions (21) concentriques s'étendent dans le sens d'axe sur toute la hauteur du bioréacteur (1) et les étages de réacteur sont reliés entre eux exclusivement par des alésages (22b), dont les axes sont agencés perpendiculairement jusqu'à tangentiellement à une tangente à la ligne périphérique de la section circulaire du bioréacteur et de façon inclinée vers le bas par rapport à l'horizontale.

6. Dispositif selon la revendication 4 ou 5, **caractérisé en ce que** les entailles (22a) ou les alésages (22b) d'insertions voisins (21) et les entailles (22a) ou alésages (22b) voisins du tuyau d'écoulement sont disposés dans le sens de rotation (29) du liquide de culture (9) décalés le plus largement possibles les uns des autres sans cependant se recouvrir.

7. Dispositif selon l'une quelconque des revendications 3 à 6, **caractérisé en ce que** le tuyau d'écoulement (18b) du bioréacteur (1) disposé en cascades est interchangeable et/ou le bioréacteur (1) disposé en cascade est subdivisé dans le sens d'axe en une partie supérieure (23) et une partie inférieure (24) reliée à l'autre partie de façon étanche, qui reçoit plusieurs récipients (28) emboîtés les uns dans les autres de façon amovible et rendus étanche de façon réciproque au moins côté fond.

8. Dispositif selon l'une quelconque des revendications 1 à 7, **caractérisé en ce que** plusieurs bioréacteurs (1) disposés sur une secoueuse à rotation (2) sont alimentés en agent nutritif par une pompe commune, de préférence une pompe à flexible multicanal (4), à partir d'un récipient de réserve (3) commun.

9. Bioréacteur pour un dispositif selon l'une quelconque des revendications 1 à 8 avec une alimentation pour des gaz, du milieu nutritif et une sortie commune pour des gaz brûlés et du liquide de culture, l'alimentation pour les gaz étant conçue comme un gazage de surface du liquide de culture, **caractérisé**
- **en ce que** la section du bioréacteur (1) est circulaire le long de son axe,
- l'axe de la sortie conçue comme un tuyau d'écoulement (18) forme un angle α de 90° à 0° par rapport à une tangente à la ligne périphérique de la section circulaire du bioréacteur (1), en particulier en formant un angle α de 0° à 10° vers l'extérieur et
- l'axe du tuyau d'écoulement (18) est agencé de façon inclinée vers le bas en formant un angle β par rapport à l'horizontale, en particulier un angle β de 2° à 45°.
